(19) Europäisches Patentamt
European Patent Office
Office européen des brevets

(11) Veröffentlichungsnummer: **0 014 433**
**B1**

(12) # EUROPÄISCHE PATENTSCHRIFT

(45) Veröffentlichungstag der Patentschrift: **18.05.83**

(21) Anmeldenummer: **80100472.2**

(22) Anmeldetag: **30.01.80**

(51) Int. Cl.³: **C 07 C 91/16,** A 61 K 31/13 // C07C101/30, C07C103/127, C07D263/04

(54) **Neues links-drehendes, basisches Derivat des 9,10-Aethanoanthracens, Verfahren zu dessen Herstellung und dieses enthaltende pharmazeutische Präparate.**

(30) Priorität: **02.02.79 CH 1046/79**
**08.01.80 CH 93/80**

(43) Veröffentlichungstag der Anmeldung: **20.08.80 Patentblatt 80/17**

(45) Bekanntmachung des Hinweises auf die Patenterteilung: **18.05.83 Patentblatt 83/20**

(84) Benannte Vertragsstaaten: **AT BE CH DE FR GB IT LU NL SE**

(56) Entgegenhaltungen: **US-A-4017542**

(73) Patentinhaber: **CIBA-GEIGY AG, Patentabteilung Postfach, CH-4002 Basel (CH)**

(72) Erfinder: **Storni, Angelo, Dr., Im Feuerbusch 3, CH-4310 Rheinfelden (CH)**

(74) Vertreter: **Zumstein, Fritz sen., Dr. et al, Bräuhausstrasse 4, D-8000 München 2 (DE)**

EP 0 014 433 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Neues links-drehendes, basisches Derivat des 9,10-Äthanoanthracens, Verfahren zu dessen Herstellung und dieses enthaltende pharmazeutische Präparate

Die vorliegende Erfindung betrifft ein neues linksdrehendes, basisches Derivat des 9,10-Äthanoanthracens und dessen Säureadditionssalze, Verfahren zur Herstellung dieser Stoffe und pharmazeutische Präparate, welche diese Stoffe enthalten.

Die erfindungsgemässe Verbindung ist das R-(−)-α-[(Methylamino)methyl]-9,10-äthanoanthracen-9(10H)-äthanol der Formel I

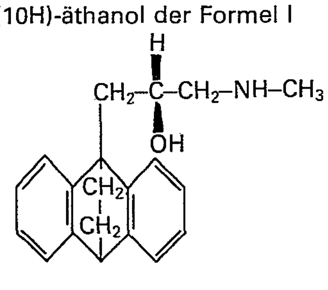

(I)

der (−)-Antipode des in der US-PS 4017542 und entsprechenden Patentschriften anderer Länder mit schweizerischer Priorität vom 23.2.1971 beschriebenen racemischen α-[(Methylamino)methyl]-9,10-äthanoantracen-9(10)-äthanol. Säureadditionssalze der Verbindung der Formel I sind insbesondere pharmazeutisch annehmbare Salze, wie das Hydrobromid, Phosphat, Methansulfonat, Äthansulfonat, 2-Hydroxyäthansulfonat, Acetat, Lactat, Malonat, Succinat, Fumarat, Maleinat, Malat, Tartrat, Citrat, Benzoat, Salicylat, Phenylacetat, Mandelat oder Embonat und vor allem das Hydrochlorid, sowie ganz allgemein gut kristallisierende Salze mit optisch aktiven Säuren, neben den bereits genannten, z. B. auch das (1:1)-Salz mit der Bis-0,0′-(p-toluoyl)-L-weinsäure. Infolge der engen Beziehungen zwischen der freien Base und ihren Säureadditionssalzen werden unter der Base und unter ihren Säureadditionssalzen sinngemäss auch die Säureadditionssalze bzw. die freie Base verstanden.

Das R-(−)-α-[(Methylamino)methyl]-9,10-äthanoanthracen-9(10H)-äthanol und seine Säureadditionsalze besitzen wertvolle pharmakologische Eigenschaften, die sich von denjenigen des bekannten Racemates und seiner Säureadditionssalze in überraschender Weise unterscheiden. Sie zeigen im Gegensatz zum bekannten Racemat in Tierversuchen praktisch keine Reserpin-antagonistische und Tetrabenazinantagonistische Wirksamkeit und wirken auch nicht hemmend auf die Noradrenalin-Aufnahme im Rattenherz und -hirn und die durch H77/77 (3-Hydroxy-4,α-dimethyl-phenäthylamin) bewirkte Noradrenadrenalin-Depletion im Rattenhirn. Dagegen besitzen die Verbindung der Formel I und ihre Säureadditionssalze sedative, antiaggressive und antihistaminische Wirksamkeit von ähnlicher Grössenordnung wie das bekannte Racemat. Bei der zur Bestimmung der sedativen Wirksamkeit gemessenen Potenzierung der durch G 29505 [2-(4-Allyl-2-methoxyphenoxy)-N,N-diäthylacetamid, [W. Theobald und R- Domenjoz, Arzneimittelforsch. 9, 285–286 (1959)] bewirkten Narkose der Maus sind die wirkungsgleichen Dosen des Hydrochlorids der Verbindung der Formel I etwa halb so gross wie diejenigen des Hydrochlorids des Racemats und somit die Verbindung der Formel I ungefähr doppelt so potent wie das Racemat, während bei der durch elektrischen Fuss-Schock ausgelösten Kampfreaktion der männlichen Mäuse [Tedeschi et al., J. Pharmacol. Exptl. Therap. 125, 28–34, (1959)] die intraperitoneal verabreichten wirkungsgleichen Dosen der Hydrochloride der Verbindung der Formel I und des Racemats ungefähr gleich gross sind und bei der Antagonisierung der Histamintoxizität am Meerschwein ebenfalls bei intraperitonealer Verabreichung das Hydrochlorid des Racemats mit einer $ED_{50}$ von etwa 0,1 mg/kg etwas wirksamer ist als das Hydrochlorid der Verbindung der Formel I mit einer $ED_{50}$ von etwa 0,25 mg/kg. Am isolierten, elektrisch stimulierten linken Vorhof von reserpinierten, d. h. mit Reserpin vorbehandelten Meerschweinchen zeigte das Hydrochlorid der Verbindung der Formel I eine ungefähr gleiche negativ inotrope Wirkung, dagegen war die negativ chronotrope Wirkung am isolierten, spontan schlagenden rechten Vorhof deutlich schwächer als diejenigen des Racemats, und der Vergleich der Wirkungen von 1 mg/ml an isolierten Vorhöfen von nicht-reserpinierten und reserpinierten Meerschweinchen zeigt, dass die Verbindung der Formel I im Gegensatz zum Racemat in dieser Konzentration nicht kardiostimulierend wirkt.

Die akute Toxizität des Hydrochlorids der Verbindung der Formel I an Mäusen und Ratten ist bei intravenöser Verabreichung ungefähr gleich gross und bei oraler Verabreichung etwas niedriger als diejenige des Hydrochlorids des Racemats. Das R-(−)-α-[(Methylamino)methyl]-9,10-äthanoanthracen-9(10H)-äthanol und seine pharmazeutisch annehmbaren Säureadditionssalze können als Wirkstoffe für Arzneimittel mit sedativer, antiaggressiver, tranquillisierender, antihistaminischer und antidepressiver Wirksamkeit, insbesondere zur Behandlung von Verstimmungszuständen ängstlicher, dysphorischer oder reizbarer Färbung und psychovegetativer oder psychosomatischer Beschwerden mit depressivem und/oder ängstlichem Hintergrund im Sinne einer larvierten Depression, Verwendung finden.

Das R-(−)-α-[(Methylamino)methyl]-9,10-äthanoanthracen-9(10H)-äthanol der Formel I und seine Säureadditionssalze werden erfindungsgemäss hergestellt, indem man

a) das racemische α-[(Methylamino)methyl]-9,10-äthanoanthracen-9(10)-äthanol auftrennt und das R-(−)-α-[(Methylamino)methyl]-9,10-äthanoanthracen-9(10H)-äthanol, gegebenenfalls in Form eines Säureadditionssalzes, isoliert, oder

b) eine Verbindung der Formel II

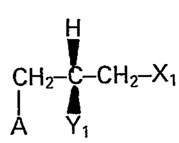

(II),

mit einer Verbindung der Formel III

$$X_2\text{–}CH_3 \quad \text{(III)}$$

umsetzt, wobei eines der Symbole $X_1$ und $X_2$ die Aminogruppe und das andere eine reaktionsfähige veresterte Hydroxygruppe und $Y_1$ eine freie Hydroxygruppe bedeutet, und $X_1$ auch zusammen mit $Y_1$ eine Epoxygruppe bedeuten kann, und A für den 9,10-Äthanoanthracen-9(10H)-yl-rest steht, oder

c) in einer Verbindung der Formel IV

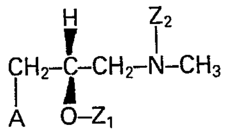

(IV)

in welcher mindestens eines der Symbole $Z_1$ und $Z_2$ einen abspaltbaren Rest und das andere gegebenenfalls Wasserstoff, oder $Z_1$ und $Z_2$ zusammen einen zweiwertigen, abspaltbaren Rest bedeuten, und A für den 9,10-Äthanoanthracen-9(10H)-yl-rest steht, den oder die Reste $Z_1$ und/oder $Z_2$ abspaltet, oder

d) eine Verbindung, welche sich von der Verbindung der Formel I nur dadurch unterscheidet, dass in ihr ein dem Stickstoffatom benachbartes Kohlenstoffatom mit letzterem durch eine Doppelbindung verbunden oder durch eine Hydroxygruppe oder durch einen Oxorest, gegebenenfalls zusammen mit Niederalkoxy, substituiert ist, reduziert, oder

e) an das R-α-[(Methylamino)methyl]-9(10H)-antracenäthanol Äthylen anlagert, oder

f) eine Verbindung der allgemeinen Formel V

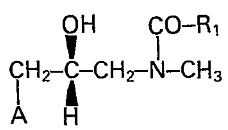

(V)

in welcher $R_1$ einen gegebenenfalls substituierten Kohlenwasserstoffrest oder einen gegebenenfalls substituierten heterocyclischen Rest und A den 9,10-Äthanoanthracen-9(10H)-yl-rest bedeutet, mit einer starken sauerstoffhaltigen anorganischen oder organischen Säure oder einem Halogenid einer solchen umsetzt und das entstandene Zwischenprodukt hydrolysiert, und gewünschtenfalls das so erhaltene R-(−)-α-[(Methylamino)methyl]-9,10-äthanoanthracen-9(10H)-äthanol in ein Säureadditionssalz überführt und/oder aus einem erhaltenen Säureadditionssalz die Base freisetzt.

Die Auftrennung und Isolierung gemäss Verfahren a) erfolgt in an sich bekannter Weise. Beispielsweise kann man das Racemat mit salzbildenden optisch aktiven Säuren, wie organischen Carbon- oder Sulfonsäuren, z. B. die (D)- und (L)-Formen von Weinsäure, Bis-0,0'-(p-toluoyl)-weinsäure, Äpfelsäure, Mandelsäure, Camphersulfonsäure, Chinasäure, Milchsäure, Glutaminsäure oder Asparaginsäure in Säureadditionssalze überführen. Die erhaltenen Gemische der entsprechenden Salze können aufgrund von physikalischchemischen Unterschieden, z. B. der Löslichkeit oder der Kristallisationsfähigkeit, in die diastereoisomeren Salze aufgetrennt und gegebenenfalls die optisch aktive (−)-Form aus dem Salz freigesetzt werden.

Aus dem Racemat kann man auch durch fraktioniertes Kristallisieren aus einem geeigneten Lösungsmittel, gegebenenfalls auch aus einem optisch aktiven Lösungsmittel, oder durch Chromatographie, insbesondere Dünnschichtchromatographie, an einem optisch aktiven Trägermaterial, die (−)-Form abtrennen.

Eine reaktionsfähige, veresterte Hydroxygruppe in einer Verbindung der Formel II oder III ist vor allem eine mit einer starken organischen oder anorganischen Säure, besonders mit einer Halogenwasserstoffsäure, wie Chlor-, Brom- oder Jodwasserstoffsäure, oder mit einer Arylsulfonsäure, wie einer durch niedere Alkyl- oder Alkoxyreste, z. B. die oben genannten, oder durch Halogenatome, wie Chlor- oder Bromatome, ein-, zwei- oder mehrfach substituierten Benzolsulfonsäure, z. B. p-Toluolsulfonsäure oder p-Brombenzolsulfonsäure, oder einer Niederalkansulfonsäure, z. B. Methansulfonsäure, veresterte Hydroxygruppe, oder, besonders als $X_2$, auch eine durch Schwefelsäure bzw. Methylschwefelsäure veresterte Hydroxygruppe. $X_1$ kann auch zusammen mit $Y_1$ als Epoxybrücke vorliegen.

Die Umsetzung gemäss b) erfolgt in üblicher Weise, vorzugsweise in Anwesenheit eines Lösungsmittels und gegebenenfalls in Gegenwart eines Kondensationsmittels, z. B. eines basischen Kondensationsmittels, vorzugsweise bei erhöhter Temperatur und gegebenenfalls im geschlossenen Gefäss unter Druck. Ein basisches Kondensationsmittel ist z. B. ein Alkalihydroxid oder -carbonat, z. B. Natriumhydroxid oder Kaliumcarbonat, oder ein tertiäres Amin, z. B. Triäthylamin oder Pyridin.

In den Ausgangsstoffen der allgemeinen Formel IV für das Verfahren c) sind abspaltbare Reste $Z_1$ und $Z_2$ ebenso wie durch $Z_1$ und $Z_2$ zusammen gebildete, zweiwertige abspaltbare Reste, beispielsweise durch Solvolyse, insbesondere Hydrolyse, oder durch Reduktion, z. B. Hydrogenolyse, abspaltbare Reste.

Als durch Solvolyse, insbesondere Hydrolyse, abspaltbare Reste $Z_1$ und $Z_2$ kommen z. B. Acylreste, wie Alkanoylreste, vor allem gegebenenfalls halogenierte, z. B. fluorierte Niederalkanoylreste, wie der Acetylrest bzw. der Trifluoracetylrest, weiter z. B. Aroyl- und Arylniederalkanoylreste, wie der Benzoyl- bzw. Phenylacetylrest, oder Acylreste von Kohlensäurehalbestern, z. B. Niederalkoxycarbonylreste, wie der Methoxycarbonyl-, Äthoxycarbonyl- oder Tert.butoxycarbonylrest, oder Aralkoxycarbonylreste, wie der Benzyloxycarbonylrest, sowie z. B. auch Silylreste, wie der Trimethylsilylrest, in Betracht.

Ein durch $Z_1$ und $Z_2$ gebildeter zweiwertiger Rest ist beispielsweise ein geminal zweiwertiger Kohlenwasserstoffrest, insbesondere ein Niederalkylidenrest, wie der Methylen-, Äthyliden- oder 1-Methyläthylidenrest (Isopropyl)-idenrest), oder

ein Aralkylidenrest wie der Benzylidenrest, ferner z. B. eine Phosphorylidengruppe, insbesondere eine Niederalkoxyphosphorylidengruppe, wie die Methoxy- oder Äthoxyphosphorylidengruppe.

Die hydrolytische Abspaltung von $Z_1$ und/oder $Z_2$ erfolgt mit hydrolysierenden Mitteln, beispielsweise in Gegenwart von sauren Mitteln, wie z. B. verdünnten Mineralsäuren, wie Schwefelsäure oder Halogenwasserstoffsäuren, besonders Salzsäure, oder bei Acylresten vorzugsweise in Gegenwart von basischen Mitteln, z. B. Alkalihydroxiden, wie Natriumhydroxid, in geeigneten organischern oder organisch-wässrigen Lösungsmitteln, in der Kälte, z. B. bei Raumtemperatur, oder vorzugsweise unter Erwärmen. Die hydrolytische Abspaltung eines durch $Z_1$ und $Z_2$ gebildeten zweiwertigen Restes kann analog erfolgen.

Durch Reduktion abspaltbare Reste $Z_1$ und $Z_2$ sind beispielsweise 1-Arylniederalkylreste, wie der Benzylrest, oder 1-Arylniederalkoxycarbonylrest, wie der Benzyloxycarbonylrest, welche beispielsweise durch Hydrogenolyse, z. B. durch Reduktion mit katalytisch aktiviertem Wasserstoff, wie Wasserstoff in Gegenwart eines Hydrierungskatalysators, wie eines Palladium- oder Platinkatalysators, abgespalten werden können. Ebenfalls durch Hydrogenolyse können durch $Z_1$ und $Z_2$ zusammen gebildete Aralkylidenreste, wie der Benzylidenrest, abgespalten werden. $Z_1$ oder $Z_2$ kann aber auch ein 2-Halogen-alkoxycarbonylrest sein, wie z. B. der 2,2,2-Trichloräthoxycarbonylrest oder der 2-Jodäthoxycarbonylrest, welche durch Reduktion abgespalten werden können. Zur Reduktion kommt vor allem die metallische Reduktion (sogenannter nascierender Wasserstoff) in Betracht, wie z. B. die Einwirkung von Metall bzw. Metallegierungen, wie auch Amalgamen, vorzugsweise in Gegenwart von Wasserstoff abgebenden Mitteln, wie Carbonsäuren, Alkoholen oder Wasser. Vor allem verwendet man Zink oder Zinklegierungen in Essigsäure. Ferner kommen auch Chrom(II)-verbindungen, wie Chrom(II)-chlorid oder Chrom(II)-acetat, in Betracht. $Z_2$ kann auch eine Arylsulfonylgruppe, wie die Toluolsulfonylgruppe, sein, die in üblicher Weise durch Reduktion mit nascierendem Wasserstoff, z. B. durch ein Alkalimetall, wie Lithium oder Natrium, in flüssigem Ammoniak abgespalten werden kann. Die Abspaltung einer Arylsulfonylgruppe kann auch mit einem Hydrid, z. B. einem der nachstehend im Zusammenhang mit dem Verfahren c) genannten einfachen oder komplexen Hydride, vorzugsweise Lithiumaluminiumhydrid, zweckmässig in Gegenwart eines inerten Lösungsmittels, wie eines ätherartigen organischen Lösungsmittels, z. B. Tetrahydrofuran, vorgenommen werden.

Ausgangsstoffe für das Verfahren d) mit einer Doppelbindung zwischen dem Stickstoffatom und einem benachbarten Kohlenstoffatom sind das R-(−)-α-[(Methylamino)methyl]-9,10-äthanoanthracen-9(10H)-äthanol und das R-α-[(Methylimino)-methyl]-9,10-äthanoanthracen-9(10H)-äthanol. Bei den in Nachbarstellung zum Stickstoffatom durch Hydroxy substituierten Verbindungen handelt es sich um das R-α-[(Hydroxymethylamino)methyl]-9,10-äthanoanthracen-9(10H)-äthanol und das R-1-(Methylamino)-9,10-äthanoanthracen-9(10H)-propan-1,2-diol. Die Reduktion der vorgenannten 4 Verbindungen kann in üblicher Weise, vorzugsweise mittels eines einfachen oder komplexen Hydrids, z. B. eines Borans, oder eines Dileichtmetallhydrids, z. B. eines Alkalimetall-erdmetallhydrids, wie Natriumborhydrid oder Lithiumaluminiumhydrid, oder eines Alkoxyaluminium- oder Alkoxyborhydrids, z. B. eines der weiter unten genannten, erfolgen.

Es ist aber auch möglich, die Reduktion als Hydrierung mit Wasserstoff in Gegenwart eines Katalysators, wie eines Platin-, Palladium- oder Nickelkatalysators, oder eines homogenen Katalysators, z. B. einer komplexen Rhodiumverbindung, wie eines Rhodium-chlor-triphenyl-phospin-komplexes, zu vollziehen.

Ist ein dem Stickstoffatom benachbartes Kohlenstoffatom durch einen Oxorest substituiert, so handelt es sich bei den entsprechenden Ausgangsstoffen einerseits um das R-N-Methyl-9,10-äthanoanthracen-9(10H)-lactamid und andrerseits um das N-[3-(9,10-Äthanoanthracen-9(10H)-yl)-2(R)-hydroxypropyl]-formamid sowie am Stickstoffatom durch den gleichen Rest substituierte Carbaminsäure-niederalkylester, wie den Methyl- und den Äthylester. Deren Reduktion kann nach den üblichen Verfahren der Amidreduktion, beispielsweise mittels eines einfachen oder komplexen Hydrids, wie eines Borans, z. B. Diboran, oder eines komplexen Dileichtmetallhydrids, insbesondere eines Alkalimetallaluminiumhydrids, wie Lithium- oder Natrium-aluminiumhydrid, in einem ätherartigen Lösungsmittel, wie Diäthyläther oder Tetrahydrofuran, oder mittels eines Alkalimetallalkoxyaluminiumhydrids oder -borhydrids, z. B. Natriumdibutoxyaluminiumhydrid oder Natrium-trimethoxyborhydrid, oder eines Erdalkalimetallaluminiumhydrids, wie Magnesium-aluminiumhydrid, oder mittels Natriumborhydrid in einem tertiären Amin, wie Pyridin oder Triäthylamin, oder mittels Aluminiumhydrid-Aluminiumchlorid erfolgen.

Die Einführung des 9,10-Äthanorestes gemäss e) kann in üblicher Weise erfolgen, z. B. durch Umsetzung des genannten Anthracenderivates mit Äthylen nach der Methode von Diels-Alder, vorteilhaft in einem geeigneten Lösungsmittel, wie einem aromatischen Kohlenwasserstoff, z. B. Benzol oder Toluol, und bei erhöhter Temperatur, wie z. B. von 5 bis 250°C, und/oder unter Druck, wie z. B. bei 2 bis 150 at.

In den Ausgangsstoffen der allgemeinen Formel V für das Verfahren f) ist ein gegebenenfalls substituierter Kohlenwasserstoffrest $R_1$, z. B. Niederalkyl, wie Äthyl, Propyl, Isopropyl, Butyl oder Tert-.butyl und vor allem Methyl, weiter z. B. Phenylniederalkyl, wie Benzyl oder 2-Phenyläthyl, oder Phenyl, wobei in diesen oder anderen Resten $R_1$ als Substituenten, z. B. Halogen bis Atomnummer 35, Niederalkyl, z. B. Methyl, Niederalkoxy, z. B. Methoxy oder Aryloxy, z. B. Phenoxy, vorliegen können. Als heterocyclischer Rest ist $R_1$, z. B. Fu-

ryl, wie 2-Furyl, Thienyl, wie 2-Thienyl, oder Pyridinyl, wie 3- oder 4-Pyridinyl.

Die entsprechenden Ausgangsstoffe der Formel V lassen sich aus dem z. B. bei der Auftrennung von racemischem α-[(Methylamino)methyl]-9,10-äthanoanthracen-9(10H)-äthanol als Nebenprodukt anfallenden freien S-(+)-α-[(Methylamino)-methyl]-9,10-äthanoanthracen-9(10H)-äthanol nach üblichen Acylierungsmethoden, insbesondere unter Verwendung von Carbonsäurehalogeniden oder Niederalkylestern oder, insbesondere für die Herstellung der als Ausgangsstoff besonders geeigneten Verbindung mit Methyl als $R_1$, von Anhydriden, wie Acetanhydrid, herstellen.

Als starke, sauerstoffhaltige anorganische oder organische Säuren kommen im Verfahren f) insbesondere konzentrierte Schwefelsäure oder Phosphorsäure, weiter z. B. starke organische Sulfonsäuren, etwa aliphatische Sulfonsäuren, z. B. Methansulfonsäure, oder aromatische Sulfonsäuren, wie eine gegebenenfalls substituierte Phenylsulfonsäure, etwa 4-Methyl-, 4-Brom-, 4-Nitro- oder 2,4-Dinitrophenylsulfonsäure oder Naphthalinsulfonsäure, z. B. 1-Naphthalinsulfonsäure; und als deren Halogenide in erster Linie die Chloride oder Bromide wie vor allem Thionylchlorid, weiter z. B. Thionylbromid, Sulfurylchlorid, Chlorsulfonsäure, Phosphortrichlorid, Phosphorpentachlorid, Phosphoroxychlorid oder Methansulfonylchlorid in Betracht. Ferner können auch den genannten Halogeniden mehrwertiger Säuren entsprechende gemischte Esterhalogenide, wie etwa ein Niederalkoxysulfonylhalogenid, z. B. Methoxy- oder Äthoxysulfonylchlorid oder Phosphorsäure-niederalkylesterhalogenide, z. B. Phosphorsäuredimethylester-chlorid verwendet werden.

Die Umsetzungen mit starken Säuren, vor allem konz. Schwefelsäure oder Phosphorsäure, werden in An- oder Abwesenheit von Lösungs- oder Verdünnungsmitteln, wie z. B. Acetanhydrid, bei Temperaturen von etwa −50 bis +200°C, und die Umsetzungen mit Säurehalogeniden, wie z. B. Thionylchlorid, ebenfalls in An- oder Abwesenheit von Lösungs- oder Verdünnungsmitteln, z. B. Kohlenwasserstoffen oder insbesondere Chlorkohlenwasserstoffen, wie Methylenchlorid, in einem Temperaturbereich von etwa −10 bis +70°C, vorzugsweise von etwa +10 bis +50°C durchgeführt. Als Reaktionsprodukte dieser Umsetzungen können 2-R-5-(9,10-äthanoantracen-9(10H)-yl)-4,5-dihydro-3-methyl-oxazolium-salze, deren Anion der zur Umsetzung verwendeten Säure entspricht bzw. im Fall von Umsetzungen mit Säurehalogeniden das entsprechende Halogenion ist, angenommen werden.

Die Hydrolyse der Zwischenprodukte wird in saurem oder basischem Medium durchgeführt. Geeignete saure Mittel sind z. B. wässrige Säuren, etwa wässrige Mineralsäuren, z. B. wässrige Salzsäure, Schwefelsäure oder Phosphorsäure. Die saure Hydrolyse wird in einem Temperaturbereich von 0 bis +120°C, zweckmässigerweise bei +10 bis +50°C durchgeführt. Als basische Medien sind z. B. wässrige Laugen, etwa die der Alkalien oder Erdalkalien wie Natriumhydroxid, oder Kaliumhydroxid, oder die Hydroxide des Calciums oder Magnesiums geeignet, wobei die genannten Reagentien vorteilhafterweise bei erhöhter Temperatur, etwa in einem Bereich von 50 bis 150°C eingesetzt werden.

Die Hydrolyse kann auch stufenweise durchgeführt werden, indem man ein Zwischenprodukt gegebenenfalls über die entsprechende freie Base als Zwischenstufe, in wässrigem Medium zu der entsprechenden N-Acylverbindung der allgemeinen Formel V mit umgekehrter sterischer Konfiguration wie die des jeweils verwendeten Ausgangsstoffes der Formel V hydrolysiert, und anschliessend diese Verbindung zu einer der Formel I entsprechenden Verbindung hydrolysiert.

Das Verfahren gemäss f) kann vorteilhafterweise auch so durchgeführt werden, dass man einen unmittelbar vorher hergestellten Ausgangsstoff der allgemeinen Formel V, ohne diesen in reiner Form zu isolieren, im gleichen Reaktionsansatz mit einer geeigneten Säure bzw. einem Halogenid einer solchen umgesetzt und das erhaltene Zwischenprodukt ebenfalls ohne weitere Reinigung der Hydrolyse unterwirft.

Die für die Verfahren b) bis e) benötigten optisch aktiven Ausgangsstoffe lassen sich entweder durch Auftrennung von bekannten racemischen, insbesondere basischen, Ausgangsstoffen in an sich bekannter Weise, oder analog zu den für die Herstellung des bekannten Racemates benötigten, racemischen Ausgangsstoffen unter Verwendung von optisch aktiven statt racemischen Vorläuferverbindungen herstellen.

Säureadditionssalze, insbesondere pharmazeutisch annehmbare Säureadditionssalze der Verbindung der Formel I, z. B. die weiter oben genannten, können in üblicher Weise hergestellt werden. Beispielsweise versetzt man eine Lösung der Base in einem organischen Lösungsmittel, wie z. B. Methylenchlorid, Äthylacetat, Äthanol oder Isopropanol, mit der als Salzkomponente gewünschten Säure oder einer Lösung derselben im gleichen oder andern organischen Lösungsmitteln, wie Äthylacetat oder Diäthyläther, und filtriert, nötigenfalls nach Abkühlen oder Einengen oder nach Zufügen eines Lösungsmittels mit schlechterem Lösungsvermögen für Salze, wie z.B. Diäthyläther, das ausgefallene Salz ab.

Die vorliegende Erfindung betrifft zudem die Verbindung der Formel I und deren pharmazeutisch annehmbare Säureadditionssalze zur Verwendung als Medikamente, insbesondere als Antidepressiva, Sedativa oder Antihistaminica, z. B. zur Anwendung bei den weiter oben genannten Indikationen, sowie ihre Verwendung zur Herstellung von entsprechenden pharmazeutischen Präparaten.

Die Dosierung der Verbindung der Formel I und ihrer pharmazeutisch annehmbaren Säureadditionssalze bei Warmblütern hängt von der Spezies, dem Körpergewicht und Alter und vom individuellen Zustand, sowie von der Applikationsweise ab. Die pro Tag verabreichten Dosen liegen zwischen etwa 0,05 und etwa 3 mg/kg, vorzugsweise zwischen etwa 0,08 und 1,5 mg/kg Körpergewicht. Durchschnittlich wird einem Warmblüter von etwa 70 kg Körpergewicht eine Tagesdosis von etwa 10 bis etwa 150 mg, vorzugsweise von etwa 30 bis etwa 75 mg Wirkstoff verabreicht.

Die vorliegende Erfindung betrifft ebenfalls pharmazeutische Präparate, welche die Verbindung der Formel I oder pharmazeutisch annehmbare Säureadditionssalze dieser Verbindung enthalten. Bei den erfindungsgemässen pharmazeutischen Präparaten handelt es sich insbesondere um solche zur enteralen, wie oralen oder rektalen, sowie zur parenteralen Verabreichung, die den pharmakologischen Wirkstoff allein oder vorzugsweise zusammen mit mindestens einem pharmazeutisch annehmbaren Trägermaterial enthalten. Solche Präparate enthalten den Wirkstoff, d. h. die Verbindung der Formel I oder ein pharmazeutisch annehmbares Säureadditionssalz derselben, in einer für die Verabreichung der oben genannten täglichen Dosen in einer oder mehreren, vorzugsweise drei, Einzeldosen geeigneten Menge und Konzentration.

Erfindungsgemäss anwendbare pharmazeutische Zusammensetzungen in Doseneinheitsformen, wie Dragées, Tabletten, Kapseln, Suppositorien oder Ampullen, enthalten als Wirkstoff pro Doseneinheit vorzugsweise 2,5–50 mg, insbesondere 5–25 mg, der Verbindung der Formel I oder vorzugsweise eines pharmazeutisch annehmbaren Säureadditionssalzes dieser Base zusammen mit mindestens einem pharmazeutischen Trägerstoff.

Doseneinheitsformen für die perorale Anwendung enthalten als Wirkstoff vorzugsweise zwischen 1 und 50% der Verbindung der Formel I oder eines pharmazeutisch annehmbaren Säureadditionssalzes derselben. Zu ihrer Herstellung kombiniert man den Wirkstoff z. B. mit festen, pulverförmigen Trägerstoffen, wie Lactose, Saccharose, Sorbit, Mannit; Stärken wie Kartoffelstärke, Maisstärke oder Amylopektin, ferner Laminariapulver oder Citruspulpenpulver; Cellulosederivaten oder Gelatine, gegebenenfalls unter Zusatz von Gleitmitteln, wie Magnesium- oder Calciumstearat oder Polyäthylenglykolen, zu Tabletten oder zu Dragée-Kernen. Die Dragée-Kernen überzieht man beispielsweise mit konzentrierten Zuckerlösungen, welche z. B. noch arabischen Gummi, Talk und/oder Titandioxid enthalten können, oder mit einem Lack, der in leichtflüssigen organischen Lösungsmitteln oder Lösungsmittelgemischen gelöst wird. Diesen Überzügen können Farbstoffe zugefügt werden, z. B. zur Kennzeichnung verschiedener Wirkstoffdosen.

Als weitere orale Doseneinheitsformen eignen sich Steckkapseln aus Gelatine sowie weiche, geschlossene Kapseln aus Gelatine und einem Weichmacher, wie Glycerin. Die Steckkapseln enthalten den Wirkstoff vorzugsweise als Granulat, z.B. in Mischung mit Füllstoffen, wie Maisstärke, und/oder Gleitmitteln, wie Talk oder Magnesiumstearat, und gegebenenfalls Stabilisatoren, wie Natriummetabisulfit ($Na_2S_2O_5$) oder Ascorbinsäure. In weichen Kapseln ist der Wirkstoff vorzugsweise in geeigneten Flüssigkeiten, wie flüssigen Polyäthylenglykolen, gelöst oder suspendiert, wobei ebenfalls Stabilisatoren zugefügt sein können.

Als erfindungsgemässe Doseneinheitsformen für die rektale Anwendung kommen z. B. Suppositorien in Betracht, welche aus einer Kombination eines Wirkstoffes mit einer Suppositoriengrundmasse bestehen. Als Suppositoriengrundmasse eignen sich z. B. natürliche oder synthetische Triglyceride, Paraffinkohlenwasserstoffe, Polyäthylenglykole oder höhere Alkanole. Ferner eignen sich auch Gelatine-Rektalkapseln, welche aus einer Kombination des Wirkstoffes mit einer Grundmasse bestehen. Als Grundmasse eignen sich z. B. flüssige Triglycerice, Polyäthylenglykole oder Paraffinkohlenwasserstoffe.

Ampullen zur parenteralen, insbesondere intramuskulären Verabreichung enthalten vorzugsweise ein wasserlösliches, pharmazeutisch annehmbares Salz der Verbindung der Formel I in einer Konzentration von vorzugsweise 0,5–5%, gegebenenfalls zusammen mit geeigneten Stabilisierungsmitteln und Puffersubstanzen, in wässriger Lösung.

Die nachfolgenden Beispiele erläutern die Herstellung der Verbindung der Formel I sowie einiger typischer Doseneinheitsformen, sollen jedoch den Umfang der Erfindung in keiner Weise beschränken.

Beispiel 1

184,8 g (0,63 Mol) racemisches α-[(Methylamino)methyl]-9,10-äthanoanthracen-9(10H)-äthanol und 127,5 g (0,315 Mol) (–)-Bis-0,0'-(p-toluoyl)-L-weinsäure werden in 2500 ml Methanol bis 40°C gelöst und anschliessend 24 h bei Raumtemperatur stehengelassen. Das ausgefallene Kristallisat wird abgenutscht und zweimal mit je 50 ml eiskaltem Methanol nachgewaschen. Das Filtrat kann gewünschtenfalls zur Gewinnung des anderen Antipoden verwendet werden. Das Nutschengut wird in 2500 ml heissem Methanol gelöst und durch Abdestillation von Methanol auf 800 ml eingeengt. Diese Lösung wird während 24 h bei Raumtemperatur stehengelassen, wobei das R-(–)-α-[(Methylamino)methyl]-9,10-äthananthracen-9(10H)-äthanol-(–)-bis-0,0'-(p-toluoyl)-L-tartrat-(1:1) auskristallisiert. Dieses wird abgenutscht, zweimal mit je 40 ml eiskaltem Methanol nachgewaschen und im Wasserstrahlvakuum bei 50°C getrocknet.
Smp. 180°C unter Zersetzung;
$[\alpha]_D^{20} = -64°C$ (c = 1,135 in Methanol).

Zur Gewinnung der freien Base löst man 4,9 g (0,01 Mol) des obigen Salzes in 50 ml Methylenchlorid, extrahiert die Lösung zweimal mit je 15 ml 1-n Natronlauge, wäscht sie hierauf zweimal mit je 15 ml Wasser und dampft sie bei ca. 14 mbar ein. Das zurückbleibende kristallierte R-(–)-α-[(Methylamino)methyl]-9,10-äthanoanthracen-9(10H)-äthanol schmilzt bei 107–108°C $[\alpha]_D^{20} = -9{,}1$ (c = 1,01 in Methanol). Gewünschtenfalls kann die Base noch aus Äther umkristallisiert werden.

Zur Herstellung des Hydrochlorids werden 56,9 g R-(–)-α-[(Methylamino)methyl]-9,10-äthanoanthracen-9(10H)-äthanol-(–)-bis-0,0'-(p-toluoyl)-L-tartrat-(1:1) in 200 ml Methylenchlorid gelöst und unter Rühren bei Raumtemperatur mit einer ätherischen Chlorwasserstofflösung versetzt, bis die überstehenden Dämpfe bleibend Kongopapier blau färben. Dabei kristallisiert R-(−)-α-[(Methylamino)methyl]-9,10-äthanoanthracen-9(10H)-äthanol-hydrochlorid aus. Nach Zugabe von 300 ml Äther wird das Kristallisat abgenutscht und anschliessend einmal aus Äthanol-Methanol umkristallisiert.

Das so gereinige Hydrochlorid schmilzt bei 231−232°C, $[\alpha]_D^{20} = -9°C$ (c = 1,37 in Methanol).

Beispiel 2

a) Zu einer Lösung von 2,93 g (0,010 Mol) S-(+)-α-[(Methylamino)methyl]-9,10-äthanoanthracen-9(10H)-äthanol in 8 ml Dimethylformamid werden bei 5−10°C 1,9 ml Acetanhydrid zugetropft. Die Lösung wird 4 h bei Raumtemperatur gerührt, dann auf 60 ml Wasser gegossen und mit 100 ml Äthylacetat extrahiert. Die Äthylacetatlösung wird mit gesättigter Natriumbicarbonatlösung gewaschen, über Natriumsulfat getrocknet und im Vakuum eingedampft. Das zurückbleibende, rohe S-(+)-α-[(N-Methylacetamido)methyl]-9,10-äthanoanthracen-9(10H)-äthanol kann direkt weiterverarbeitet werden.

b) Das Rohprodukt von a (2,5 g) wird in 6 ml Methylenchlorid gelöst. Unter Rühren wird bei 5−10°C innerhalb 15 min eine Lösung von 0,77 ml Thionylchlorid in 4 ml Methylenchlorid zugegeben. Die Reaktionslösung wird 2 h bei 20°C und dann eine Stunde bei 35°C gerührt und anschliessend bei ca. 14 mbar (Wasserstrahlvakuum) zur Trockne eingedampft.

c) Der Eindampfrückstand von b) wird in 10 ml Äthanol gelöst, mit 2,5 ml Wasser und 1,6 g Natriumhydroxid versetzt und 3 h unter Rückfluss gekocht. Dann wird das Reaktionsgemisch bei ca. 14 mbar eingeengt, mit 50 ml Eiswasser versetzt und dann mit 100 ml Äthylacetat extrahiert. Die Äthylacetatlösung wird mit Wasser neutral gewaschen, über Natriumsulfat getrocknet und bei ca. 14 mbar eingedampft, wobei die rohe, invertierte Base zurückbleibt.

Der Rückstand wird in 10 ml Methylenchlorid gelöst und mit ätherischer Chlorwasserstofflösung versetzt, wobei das R-(−)-α-[(Methylamino)-methyl]-9,10-äthanoanthracen-9(10H)-äthanol-hydrochlorid auskristallisiert. Die Kristalle werden abgenutscht und aus Isopropanol umkristallisiert. Smp. 229−231°C, $[\alpha]_D^{20} = -9 \pm 1°C$ (c = 1,6 in Methanol).

Der Ausgangsstoff für Abschnitt a) wird wie folgt hergestellt:

Das im Beispiel 1 erwähnte, den Antipoden enthaltende Filtrat wird bei etwa 14 mbar eingedampft.

Der Rückstand wird in 500 ml Methylenchlorid gelöst und diese Lösung dreimal mit je 100 ml 2-n Natronlauge und dann noch zweimal mit je 100 ml Wasser ausgeschüttelt. Nach Abdampfen des Methylenchlorids erhält man 132,5 g partiell an S-(+)-α-[(Methylamino)methyl]-9,10-äthanoanthracen-9(10H)-äthanol angereicherte Base. Diese Base (0,452 Mol) und 91,4 g (0,226 Mol) (+)-Bis-0,0′-(p-toluoyl)-D-Weinsäure werden in 1800 ml Methanol bei 40°C gelöst und dann 24 h bei Raumtemperatur stehen gelassen. Das ausgefallene Kristallisat wird abgenutscht, in Methanol gelöst, die erhaltene Lösung auf etwa ein Drittel ihres Volumens eingeengt und wiederum 24 h bei Raumtemperatur stehengelassen. Die ausgeschiedenen Kristalle werden abgenutscht und mit wenig kaltem Methanol nachgewaschen, wobei man das S-(+)-α-[(Methylamino)methyl]-9,10-äthanoanthracen-9(10H)-äthanol-(+)-bis-0,0′-(p-toluoyl)-D-tartrat-(1:1) erhält, das bei 178°C unter Zersetzung schmilzt; $[\alpha]_D^{20} = +63°C$ (c = 0,774 in Methanol). Zur Freisetzung der Base werden 88,4 g (0,18 Mol) des obigen Salzes in 500 ml Methylenchlorid gelöst und diese Lösung dreimal mit je 100 ml 2-n Natronlauge und dann noch zweimal mit je 100 ml Wasser ausgeschüttelt. Das nach Abdampfen des Methylenchlorid zurückbleibende S-(+)-α-[(Methylamino)methyl]-9,10-äthanoanthracen-9(10H)-äthanol (Smp. 106−107°C) kann direkt für die Acetylierung gemäss a) verwendet werden.

Beispiel 3

2,93 g (0,010 Mol) S-(+)-α-[(Methylamino)methyl]-9,10-äthanoanthracen-9(10H)-äthanol werden portionenweise in 14 ml Acetanhydrid gelöst. Diese Lösung versetzt man unter Rühren mit einer Lösung von 1,75 g 96%iger Schwefelsäure in 6 ml Acetanhydrid und kocht dann das Gemisch 3 h unter Rückfluss. Anschliessend wird die erhaltene Lösung bei ca. 14 mbar eingeengt und das zurückbleibende, durch N-Acetylierung und Cyclisierung entstandene Reaktionsprodukt in 30 ml 1-n Schwefelsäure aufgenommen und 2 h unter Rückfluss gekocht. Dann werden 50 g Eis zugegeben und das erhaltene Gemisch mit wässriger Ammoniaklösung auf pH 9 eingestellt und zweimal mit je 50 ml Äthylacetat extrahiert. Die vereinigten organischen Phasen werden über Natriumsulfat getrocknet und zur Trockne eingedampft. Die zurückbleibende rohe Base wird in 10 ml Methylenchlorid gelöst und mit ätherischer Chlorwasserstofflösung versetzt, wobei das R-(−)-α-[(Methylamino)methyl]-9,10-äthanoanthracen-9(10H)-äthanol-hydrochlorid auskristallisiert. Es wird abgenutscht und aus Isopropanol umkristallisiert; Smp. 228−230°C.

Beispiel 4

3,9 g des gemäss a) erhaltenen, rohen 5-(R)-[(9,10-Äthanoanthracen-9(10H)-yl)methyl]-3-methyl-oxazolidin werden mit 60 ml 2-n Salzsäure 3 h auf 90°C erwärmt. Anschliessend gibt man 5-n Natronlauge bis zur alkalischen Reaktion zu, extahiert mit Methylenchlorid und dampft die organische Phase ein. Das zurückbleibende, rohe R-α-(−)-[(Methylamino)methyl]-9,10-äthanoanthracen-9(10H)-äthanol wird in 10 ml Äthanol gelöst und 1 ml einer 10%igen äthanolischen Chlorwasserstofflösung zugefügt. Durch Versetzen mit Äther wird das Hydrochlorid der obigen Base zur Kristallisation gebracht. Das kristallisierte Hydrochlorid wird abgenutscht, gewünschtenfalls analog Beispiel 1 oder Beispiel 2 weiter gereinigt.

Der Ausgangsstoff kann wie folgt hergestellt werden:

a) 20,0 g R-α-[Aminomethyl)-9,10-äthanoanthracen-9(10H)-äthanol (aus der entsprechenden, in der US-PS 4017542, Beispiel 1, beschriebenen racemischen Verbindung vom Smp. 176–177°C, z. B. ähnlich vorliegendem Beispiel 1 erhältlich) werden in einem Gemisch von 10 ml 35%iger wässriger Formaldehydlösung und 150 ml Ameisensäure eine Stunde auf 95°C erhitzt. Das Reaktionsgemisch wird im Vakuum eingedampft, der Rückstand durch Zugabe von 2-n Natronlauge alkalisch gestellt und mit Methylenchlorid extrahiert. Die organische Phase wird eingedampft, wobei das 5(R)-[(9,10-äthanoanthracen-9(10H)-yl)-methyl]-3-methyl-oxazolidin zurückbleibt.

Beispiel 5

Zu einer Suspension von 0,7 g Lithiumaluminiumhydrid in 20 ml Tetrahydrofuran gibt man eine Lösung von 1,5 g N-[3-(9,10-Äthanoanthracen-9(10H)-yl)-2(R)-hydroxypropyl]-formamid in 20 ml Tetrahydrofuran und kocht das Gemisch 4 Stunden unter Rückfluss. Anschliessend wird es abgekühlt und mit 1,4 ml Wasser, dann 1,4 ml 15%iger Natronlauge und nochmals 5 ml Wasser versetzt. Den ausgefallenen Niederschlag filtriert man ab, dampft das Filtrat ein und löst den Rückstand in 2-n Essigsäure. Die saure Lösung wird mit Äther gewaschen, hierauf mit 10%iger Natronlauge bis zur alkalischen Reaktion versetzt und mit Methylenchlorid extrahiert. Das Lösungsmittel wird abgedampft und das zurückbleibende, rohe R-(−)-α-[(Methylamino)methyl]-9,10-äthanoanthracen-9(10H)-äthanol analog Beispiel 1 oder 2 in das Hydrochlorid vom Smp. 231–232 übergeführt.

Das als Ausgangsmaterial verwendete, substituierte Formamid kann wie folgt hergestellt werden:

a) 5 g (R)-α-(Aminomethyl)-9,10-äthanoanthracen-9(10H)-äthanol (vgl. Beispiel 4a) werden in 75 ml Ameisensäureäthylester 2 h unter Rückfluss gekocht. Die erkaltete Lösung wird bei ca. 14 mbar zur Trockne eingedampft. Der Rückstand wird in 75 ml Methylenchlorid gelöst, diese Lösung mit 40 ml 1-n Salzsäure gewaschen, über Natriumsulfat getrocknet und wiederum bei ca. 14 mbar zur Trockene eingedampft. Das zurückbleibende N-[3-(9,10-Äthanoanthracen-9(10H)-yl)-2(R)-hydroxypropyl]-formamid kann direkt für die Reduktion verwendet werden.

Beispiel 6

Eine Lösung von 10 g R-α-[(Methylamino)methyl]anthracen-9(10H)-äthanol in 200 ml Benzol wird im Autoklaven mit Äthylen unter einem Druck von 70 at. 6 h auf 70°C erwärmt. Anschliessend extrahiert man mit 200 ml 2-n Salzsäure. Der saure Extrakt wird alkalisch gestellt und mit Methylenchlorid extrahiert. Die organische Phase wird eingedampft und das zurückbleibende, rohe R-α-(−)-[(Methylamino)methyl]-9,10-äthanoanthracen-9(10H)-äthanol analog Beispiel 1 in sein Hydrochlorid übergeführt.

Beispiel 7

a) 100 g R-(−)-α-[(Methylamino)methyl]-9,10-äthanoanthracen-9(10H)-äthanol-hydrochlorid werden mit 202 g Lactose und 195 g Kartoffelstärke vermischt, die Mischung mit einer alkoholischen Lösung von 10 g Stearinsäure befeuchtet und durch ein Sieb granuliert. Nach dem Trocknen mischt man 200 g Kartoffelstärke, 250 g Talk, 3,0 g Magnesiumstearat und 40 g kolloidales Siliciumdioxid zu und presst die Mischung zu 10000 Tabletten von je 100 mg Gewicht und 10 mg Wirkstoffgehalt, die gewünschtenfalls mit Teilkerben zur feineren Anpassung der Dosierung versehen sein können.

b) Aus 50 g R-(−)-α-[(Methylamino)methyl]-9,10-äthanoanthracen-9(10H)-äthanol-hydrochlorid, 228,40 g Lactose und der alkoholischen Lösung von 7,5 g Stearinsäure stellt man ein Granulat her, das man nach dem Trocknen mit 56,60 g kolloidalem Siliciumdioxid, 200 g Talk, 20 g Kartoffelstärke und 2,50 g Magnesiumstearat mischt und zu 10000 Dragée-Kernen presst. Diese werden anschliessend mit einem konzentrierten Sirup aus 417,3 g krist. Saccarose, 6 g Schellack, 10 g arabischem Gummi, 0,2 g Farbstoff und 1,5 g Titandioxid überzogen und getrocknet. Die erhaltenen Dragées wiegen je 120 mg und enthalten je 5 mg Wirkstoff.

c) Um 1000 Kapseln mit je 10 mg Wirkstoffgehalt herzustellen, mischt man 10 g R-(−)-α-[(Methylamino)methyl]-9,10-äthanoanthracen-9(10H)-äthanol-hydrochlorid mit 263 g Lactose, befeuchtet die Mischung gleichmässig mit einer wässrigen Lösung von 2 g Gelatine und granuliert sie durch ein geeignetes Sieb (z. B. Sieb III nach Ph. Helv. V). Das Granulat mischt man mit 10 g getrockneter Maisstärke und 15 g Talk und füllt es gleichmässig in 1000 Hartgelatinekapseln der Grösse 1.

d) Man bereitet eine Suppositoriengrundmasse aus 2,0 g R-(−)-α-[(Methylamino)methyl]-9,10-äthanoanthracen-9(10H)-äthanol-hydrochlorid und 168,0 g Adeps solidus und giesst damit 100 Suppositorien mit je 20 mg Wirkstoffgehalt.

e) Eine Lösung von 25 g R-(−)-α-[(Methylamino)methyl]-9,10-äthanoanthracen-9(10H)-äthanol-hydrochlorid in einem Liter Wasser wird in 1000 Ampullen abgefüllt und sterilisiert. Eine Ampulle enthält eine 2,5%ige Lösung von 25 mg Wirkstoff.

**Patentansprüche**
**für die Vertragsstaaten BE, CH, DE, FR, GB, IT, LU, NL, SE**

1. R-(−)-α-[(Methylamino)methyl]-9,10-äthanoanthracen-9(10H)-äthanol der Formel I

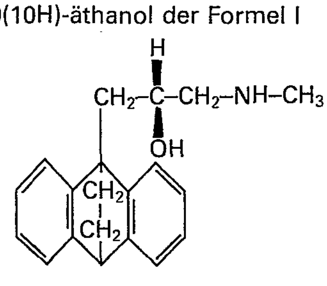

(I)

und seine Säureadditionssalze.

2. Die pharmazeutisch annehmbaren Säureadditionssalze der Verbindung der im Anspruch 1 angegebenen Formel I.

3. Das Hydrochlorid der Verbindung der im Anspruch 1 angegebenen Formel I.

4. Verfahren zur Herstellung des R-(−)-α-[(Methylamino)methyl]-9,10-äthanoanthracen-9(10H)-äthanol der im Anspruch 1 angegebenen Formel I und seiner Säureadditionssalze, dadurch gekennzeichnet, dass man

a) das racemische α-[(Methylamino)methyl]-9,10-äthanoanthracen-9(10H)-äthanol auftrennt und das R-(−)-α-[(Methylamino)methyl]-9,10-äthanoanthracen-9(10H)-äthanol, gegebenenfalls in Form eines Säureadditionssalzes, isoliert, oder

b) eine Verbindung der Formel II

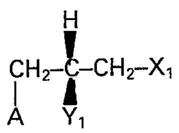

(II),

mit einer Verbindung der Formel III

$$X_2\text{–}CH_3 \qquad \text{(III)}$$

umsetzt, wobei eines der Symbole $X_1$ und $X_2$ die Aminogruppe und das andere eine reaktionsfähige veresterte Hydroxygruppe und $Y_1$ eine freie Hydroxygruppe bedeutet, und $X_1$ auch zusammen mit $Y_1$ eine Epoxygruppe bedeuten kann, und A für den 9,10-Äthanoanthracen-9(10H)-yl-rest steht, oder

c) in einer Verbindung der Formel IV

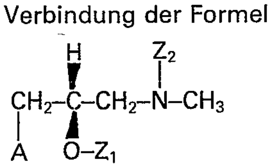

(IV)

in welcher mindestens eines der Symbole $Z_1$ und $Z_2$ einen abspaltbaren Rest und das andere gegebenenfalls Wasserstoff, oder $Z_1$ und $Z_2$ zusammen einen zweiwertigen, abspaltbaren Rest bedeuten, und A für den 9,10-Äthanoanthracen-9(10H)-yl-rest steht, den oder die Reste $Z_1$ und/oder $Z_2$ abspaltet, oder

d) eine Verbindung, welche sich von der Verbindung der Formel I nur dadurch unterscheidet, dass in ihr ein dem Stickstoffatom benachbartes Kohlenstoffatom mit letzterem durch eine Doppelbindung verbunden oder durch eine Hydroxygruppe oder durch einen Oxorest, gegebenenfalls zusammen mit Niederalkoxy, substituiert ist, reduziert, oder

e) an das R-α-[(Methylamino)methyl]-9(10H)-anthracen-äthanol Äthylen anlagert, oder

f) eine Verbindung der allgemeinen Formel V

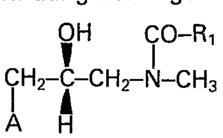

(V)

in welcher R einen gegebenenfalls substituierten Kohlenwasserstoffrest oder einen gegebenenfalls substituierten heterocyclischen Rest und A den 9,10-Äthanoanthracen-9(10H)-yl-rest bedeutet, mit einer starken sauerstoffhaltigen, anorganischen oder organischen Säure oder einem Halogenid einer solchen umsetzt und das entstandene Zwischenprodukt hydrolysiert, und gewünschtenfalls das so erhaltene R-(−)-α-[(Methylamino)methyl]-9,10-äthanoanthracen-9(10H)-äthanol in ein Säureadditionssalz überführt und/oder aus einem erhaltenen Säureadditionssalz die Base freisetzt.

5. Pharmazeutische Präparate, gekennzeichnet durch einen Gehalt an der Verbindung der im Anspruch 1 angegebenen Formel I oder einem pharmazeutisch annehmbaren Säureadditionssalz derselben, und mindestens einem pharmazeutischen Trägerstoff.

6. Pharmazeutische Präparate gemäss Anspruch 5 in Doseneinheitsform, gekennzeichnet durch einen Gehalt pro Doseneinheit von 2,5 bis 50 mg der Verbindung der in Anspruch 1 angegebenen Formel I oder eines pharmazeutisch annehmbaren Säureadditionssalzes derselben.

7. Pharmazeutische Präparate gemäss Anspruch 5 in Doseneinheitsform, gekennzeichnet durch einen Gehalt pro Doseneinheit von 5 bis 25 mg der Verbindung der in Anspruch 1 angegebenen Formel I oder eines pharmazeutisch annehmbaren Säureadditionssalzes derselben.

8. Pharmazeutische Präparate gemäss einem der Ansprüche 5, 6 und 7, gekennzeichnet durch einen Gehalt an dem Hydrochlorid der Verbindung der im Anspruch 1 angegebenen Formel I.

9. Die Verbindung der im Anspruch 1 angegebenen Formel I oder ein pharmazeutisch annehmbares Säureadditionssalz derselben zur Anwendung als Sedativum.

10. Die Verbindung der im Anspruch 1 angegebenen Formel I oder ein pharmazeutisch annehmbares Säureadditionssalz derselben zur Anwendung als Antihistaminicum.

11. Die Verbindung der im Anspruch 1 angegebenen Formel I oder ein pharmazeutisch annehmbares Säureadditionssalz derselben zur Anwendung als Antidepressivum.

12. Verwendung der Verbindung der im Anspruch 1 angegebenen Formel I oder eines pharmazeutisch annehmbaren Säureadditionssalzes derselben zur Herstellung von pharmazeutischen Präparaten mit sedativer, antihistaminischer und antidepressiver Wirksamkeit.

**Patentansprüche für den Vertragsstaat AT**

1. Verfahren zur Herstellung des R-(−)-α-[(Methylamino)methyl]-9,10-äthanoanthracen-9(10H)-äthanol der Formel I

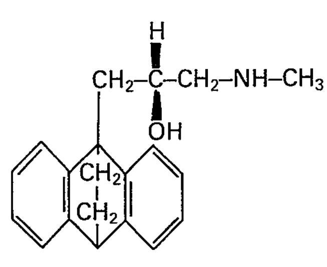

(I)

und seiner Säureadditionssalze, dadurch gekennzeichnet, dass man

a) das racemische α-[(Methylamino)methyl]-9,10-äthanoanthracen-9(10H)-äthanol auftrennt und das R-(−)-α-[(Methylamino)methyl]-9,10-äthanoanthracen-9(10H)-äthanol, gegebenenfalls in Form eines Säureadditionssalzes, isoliert, oder

b) eine Verbindung der Formel II

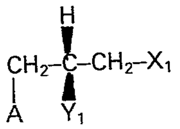

(II),

mit einer Verbindung der Formel III

$$X_2-CH_3 \qquad (III)$$

umsetzt, wobei eines der Symbole $X_1$ und $X_2$ die Aminogruppe und das andere eine reaktionsfähige veresterte Hydroxygruppe und $Y_1$ eine freie Hydroxygruppe bedeutet, und $X_1$ auch zusammen mit $Y_1$ eine Epoxygruppe bedeuten kann, und A für den 9,10-Äthanoanthracen-9(10H)-yl-rest steht, oder

c) in einer Verbindung der Formel IV

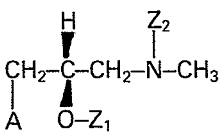

(IV)

in welcher mindestens eines der Symbole $Z_1$ und $Z_2$ einen abspaltbaren Rest und das andere gegebenenfalls Wasserstoff, oder $Z_1$ und $Z_2$ zusammen einen zweiwertigen, abspaltbaren Rest bedeuten, und A für den 9,10-Äthanoanthracen-9(10H)-yl-rest steht, den oder die Reste $Z_1$ und/oder $Z_2$ abspaltet, oder

d) eine Verbindung, welche sich von der Verbindung der Formel I nur dadurch unterscheidet, dass in ihr ein dem Stickstoffatom benachbartes Kohlenstoffatom mit letzterem durch eine Doppelbindung verbunden oder durch eine Hydroxygruppe oder durch einen Oxorest, gegebenenfalls zusammen mit Niederalkoxy, substituiert ist, reduziert, oder

e) an das R-α-[(Methylamino)methyl]-9(10H)-anthracen-äthanol Äthylen anlagert, und gewünschtenfalls das so erhaltene R-(-)-α[Methylamino)methyl]-9,10-äthano-anthracen-9(10H)-äthanol in ein Säureadditionssalz überführt oder aus einem erhaltenen Säureadditionssalz die Base freisetzt.

2. Abänderung des Verfahrens gemäss Anspruch 1, dadurch gekennzeichnet, dass man eine Verbindung der allgemeinen Formel V

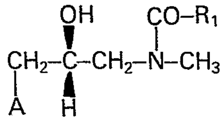

(V)

in welcher R einen gegebenenfalls substituierten Kohlenwasserstoffrest oder einen gegebenenfalls substituierten heterocyclischen Rest und A den 9,10-Äthanoanthracen-9(10H)-yl-rest bedeutet, mit einer starken sauerstoffhaltigen, anorganischen oder organischen Säure oder einem Halogenid einer solchen umsetzt und das entstandene Zwischenprodukt hydrolysiert, und gewünschtenfalls das so erhaltene R-(−)-α-[(Methylamino)methyl]-9,10-äthanoanthracen-9(10H)-äthanol in ein Säureadditionssalz überführt oder aus einem erhaltenen Säureadditionssalz die Base freisetzt.

3. Verfahren nach Anspruch 1, dadurch gekennzeichnet, dass man ein pharmazeutisch annehmbares Säureadditionssalz des R-(−)-α-[(Methylamino)methyl]-9,10-äthanoanthracen-9(10H)-äthanols herstellt.

4. Verfahren nach Anspruch 1, dadurch gekennzeichnet, dass man das Hydrochlorid des R-(−)-α-[(Methylamino)methyl]-9,10-äthanoanthracen-9(10H)-äthanols herstellt.

**Claims**
**(for the Contracting states: BE, CH, DE, FR, GB, IT, LU, NL, SE)**

1. R-(−)-α-[(Methylamino)methyl]-9,10-ethanoanthracene-9(10H)-ethanol of the formula I

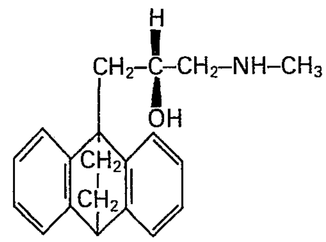

(I)

or an acid addition salt thereof.

2. A pharmaceutically acceptable acid addition salt of the compound of the formula I as indicated in claim 1.

3. The hydrochloride of the compound of the formula I as indicated in claim 1.

4. A process for the production of R-(−)-α-[(methylamino)methyl]-9,10-ethanoanthracene-9(10H)-ethanol of the formula I as indicated in claim 1, or of an acid addition salt thereof, which process comprises.

a) resolving the racemic α-[(methylamino)-methyl]-9,10-ethanoanthracene-9(10H)-ethanol and isolating R-(−)-α-[(methylamino)methyl]-9,10-ethanoanthracene-9(10H)-ethanol, if desired in the form of an acid addition salt, or

b) reacting a compound of the formula II

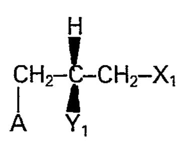

(II),

with a compound of the formula III

$$X_2-CH_3 \qquad (III)$$

wherein one of $X_1$ and $X_2$ is the amino group and the other is a reactive esterified hydroxyl group, and $Y_1$ is a free hydroxyl group, and $X_1$ together with $Y_1$ can also be an epopxy group, and A is the 9,10-ethanoanthracen-9(10H)-yl-radical, or

c) in a compound of the formula IV

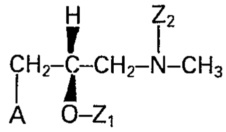

(IV)

in which at least one of $Z_1$ and $Z_2$ is a removable radical and the other may be hydrogen, or $Z_1$ and $Z_2$ together are a divalent removable radical, and A is the 9,10-ethanoanthracen-9(10H)-yl radical, removing $Z_1$ and/or $Z_2$,

d) reducing a compound which differs from the compound of the formula I only in that, in said compound, a carbon atom adjacent to the nitrogen atom is attached to this latter through a double bond or is substituted by a hydroxyl group or an oxo radical, optionally together with lower alkoxy, or

e) adding ethylene to R-α-[(methylamino)methyl]-9(10H)-anthracene-ethanol, or

f) reacting a compound of the general formula V

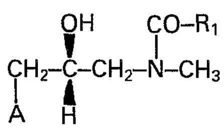

(V)

in which R is an unsubstituted or substituted hydrocarbon radical or an unsubstituted or substituted heterocyclic radical, and A is the 9,10-ethanoanthracen-9(10)-yl radical, with a strong oxygen-containing inorganic or organic acid or with a halide thereof, and hydrolysing the intermediate obtained, and, if desired, converting the resultant R-(−)-α-[(methylamino)methyl]-9,10-ethanoanthracene-9(10H)-ethanol into an acid addition salt and/or liberating the base from a resultant acid addition salt.

5. A pharmaceutical composition containing a compound of the formula I as indicated in claim 1, or a pharmaceutically acceptable acid addition salt thereof, and at least one pharmazeutical carrier.

6. A pharmaceutical composition according to claim 5 in dosage unit form, which contains, per dosage unit, from 2.5 to 50 mg of the compound of the formula I as indicated in claim 1, or of a pharmaceutically acceptable acid addition salt thereof.

7. A pharmaceutical composition according to claim 5 in dosage unit form, which contains, per dosage unit, from 5 to 25 mg of the compound of the formula I as indicated in claim 1, or of a pharmaceutically acceptable acid addition salt thereof.

8. A pharmaceutical composition according to any one of claims 5, 6 or 7, which contains the hydrochloride of the compound of the formula I as indicated in claim 1.

9. Use of the compound of the formula I as indicated in claim 1, or of a pharmaceutically acceptable acid addition salt thereof, as sedative.

10. Use of the compound of the formula I as indicated in claim 1, or of a pharmaceutically acceptable acid addition salt thereof, as antihistamine.

11. Use of the compound of the formula I as indicated in claim 1, or of a pharmaceutically acceptable acid addition salt thereof, as antidepressant.

12. Use of the compound of the formula I as indicated in claim 1, or of a pharmaceutically acceptable acid addition salt thereof, for the preparation of a pharmaceutical composition having sedative, antihistaminic or antidepressive properties.

**Revendications**
**(pour les Etats contractants: BE, CH, DE, FR, GB, IT, LU, NL, SE)**

1. R-(−)-α-[(méthylamino)méthyl]-9,10-éthanoanthracène-9(10H)-éthanol de formule I

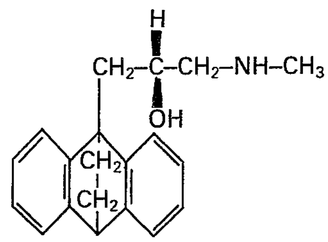

(I)

et ses sels d'addition d'acides.

2. Les sels d'addition d'acides pharmaceutiquement acceptables du composé de formule I donnée dans la revendication 1.

3. Le chlorhydrate du composé de la formule I donnée dans la revendication 1.

4. Procédé de préparation du R-(−)-α-[(méthylamino)méthyl]-9,10-éthanoanthracène-9(10H)-éthanol de formule I donnée dans la revendication 1 et de ses sels d'addition d'acides, caractérisé en ce que

a) on sépare 1'-α-[(méthylamino)méthyl]-9,10-éthanoanthracène-9(10H)-éthanol racémique et on isole le R-(−)-α-[(méthylamino)méthyl]-9,10-éthanoanthracène-9(10H)-éthanol, éventuellement sous la forme d'un sel d'addition d'acide, ou

b) on fait réagir un composé de formule II

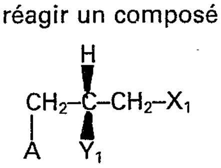

(II),

avec un composé de formule III

$$X_2\text{–}CH_3 \qquad \text{(III)}$$

où l'un des symboles $X_1$ et $X_2$ représente le groupe amino et l'autre un groupe hydroxy estérifié réactif et $Y_1$ un groupe hydroxy libre, et $X_1$ peut également représenter avec $Y_1$ un groupe époxy, et A représente le radical 9,10-éthanoanthracène-9(10H)-yle, ou

c) dans un composé de formule IV

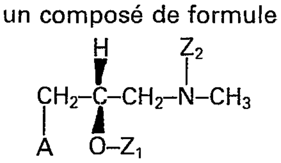

(IV)

où au moins l'un des symboles $Z_1$ ou $Z_2$ repré-

sente un radical séparable et l'autre éventuellement un hydrogène, ou bien où $Z_1$ et $Z_2$ forment ensemble un radical bivalent séparable, et A représente le radical 9,10-éthanoanthracène-9(10H)-yle, on sépare le ou les radicaux $Z_1$ et/ou $Z_2$, ou

d) on réduit un composé qui ne se différencie du composé de formule I qu'en ce qu'un atome de carbone voisin de l'atome d'azote est relié avec celui-ci par une double liaison ou est substitué par un groupe hydroxy ou par un radical oxo, éventuellement avec un alcoxy inférieur, ou

e) on fixe de l'éthylène sur le R-α-[(méthylamino)méthyl]-9(10H)-anthracène-éthanol, ou

f) on fait réagir un composé de formule V

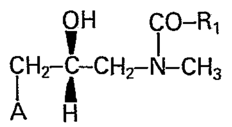
(V)

où R représente un radical hydrocarboné éventuellement substitué ou un radical hétérocyclique éventuellement substitué et A représente un radical 9,10-éthanoanthracène-9(10H)-yle, avec un acide organique ou inorganique fort contenant de l'oxygène ou un halogénure d'un tel acide, et on hydrolyse le produit intermédiaire apparu, et si on le désire on transforme le R-(−)-α-[(méthylamino)-méthyl]-9,10-éthanoanthracène-9(10H)-éthanol ainsi obtenu en un sel d'addition d'acide et/ou on libère le base à partir d'un sel d'addition d'acide obtenu.

5. Préparations pharmaceutiques caractérisées en ce qu'elles contiennent un composé de formule I donnée dans la revendication 1 ou un de ses sels d'addition d'acides pharmaceutiquement acceptables, et au moins un support pharmaceutique.

6. Préparations pharmaceutiques selon la revendication 5 sous forme posologique unitaire, caractérisée en ce qu'elles contiennent par unité posologique de 2,5 à 50 mg du composé de formule I donné dans la revendication 1 ou d'un de ses sels d'addition d'acides pharmaceutiquement acceptables.

7. Préparations pharmaceutiques selon la revendication 5 sous forme posologique unitaire, caractérisées en ce qu'elles contiennent par unité posologique de 5 à 25 mg du composé de formule I donné dans la revendication 1 ou d'un de ses sels d'addition d'acides pharmaceutiquement acceptables.

8. Préparations pharmaceutiques selon l'une des revendications 5, 6 et 7, caractérisées en ce qu'elles contiennent le chlorhydrate du composé de formule I donnée dans la revendication 1.

9. Composé de formule I donnée dans la revendication 1 ou un de ses sels d'addition d'acides pharmaceutiquement acceptables aux fins d'application comme sédatif.

10. Composé de formule I donnée dans la revendication 1 ou un de ses sels d'addition d'acides pharmaceutiquement acceptables aux fins d'application comme anti-histaminique.

11. Composé de formule I donnée dans la revendication 1 ou un de ses sels d'addition d'acides pharmaceutiquement acceptables aux fins d'application comme agent anti-dépresseur.

12. Application du composé de formule I donnée dans la revendication 1 ou d'un de ses sels d'addition d'acides pharmaceutiquement acceptables à la préparation de préparations pharmaceutiques à action sédative, anti-histaminique et anti-dépressive.

**Claims for the designated state: AT**

1. A process for the production of R-(−)-α-[(methylamino)methyl]-9,10-ethanoanthracene-9(10H)-ethanol of the formula I or of an acid addition salt thereof,

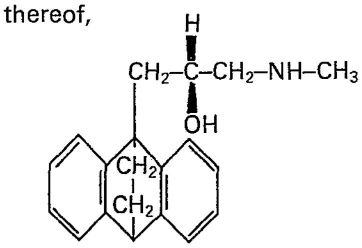
(I)

which process comprises

a) resolving the racemic α-[(methylamino)-methyl]-9,10-ethanoanthracene-9(10H)-ethanol and isolating R-(−)-α-[(methylamino)methyl]-9,10-ethanoanthracene-9(10)-ethanol, if desired in the form of an acid addition salt, or

b) reacting a compound of the formula II

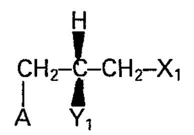
(II),

with a compound of the formula III

$$X_2\text{–}CH_3 \qquad \text{(III)}$$

wherein one of $X_1$ and $X_2$ is the amino group and the other is a reactive esterified hydroxyl group, and $Y_1$ is a free hydroxyl group, and $X_1$ together with $Y_1$ can also be an epoxy group, and A is the 9,10-ethanoanthracene-9(10H)-yl radical, or

c) in a compound of the formula IV

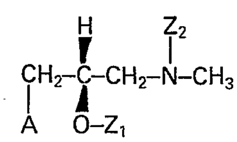
(IV)

in which at least one of $Z_1$ and $Z_2$ is a removable radical and the other may be hydrogen, or $Z_1$ and $Z_2$ together are a divalent removable radical, and A is the 9,10-ethanoanthracene-9(10H)- yl radical, removing $Z_1$ and/or $Z_2$.

d) reducing a compound which differs from the compound of the formula I only in that, in said compound, a carbon atom adjacent to the nitrogen atom is attached to this latter through a double bond or is substituted by a hydroxyl group or an oxo radical, optionally together with lower alkoxy, or

e) adding ethylene to R-α-[(methylamino)-methyl]-9(10H)-anthracene-ethanol, and, if desired, converting the resultant R-(−)-α-[(methylamino)methyl]-9,10-ethanoanthracene-9(10H)-ethanol into an acid addition salt and/or liberating the base from a resultant acid addition sald.

2. A modification of the process according to claim 1, which comprises reacting a compound of the general formula V

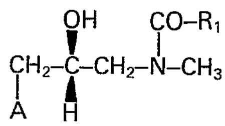 (V)

in which $R_1$ is an unsubstituted or substituted hydrocarbon radical or an unsubstituted or substituted heterocyclic radical, and A is the 9,10-ethanoanthracene-9(10H)-yl radical, with a strong oxygen-containing inorganic or organic acid or with a halide thereof, and hydrolysing the intermediate obtained, and, if desired, converting the resultant R-(−)-α-[(methylamino)methyl]-9,10-ethanoanthracene-9(10H)-ethanol into an acid addition salt and/or liberating the base from a resultant acid addition salt.

3. A process according to claim 1 for the production of a pharmaceutically acceptable acid addition salt of R-(−)-α-[(methylamino)methyl]-9,10-ethanoanthracene-9(10H)-ethanol.

4. A process according to claim 1 for the production of the hydrochloride of R-(−)-α-[(methylamino)methyl]-9,10-ethanoanthracene-9(10H)-ethanol.

**Revendications pour l'Etat contractant: AT**

1. Procédé de préparation du R-(−)-α-[méthylamino)méthyl]-9,10-éthanoanthracène-9(10H)-éthanol de formule I

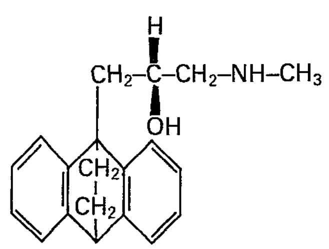 (I)

et de ses sels d'addition d'acides, caractérisé en ce que

a) on sépare 1'α-[(méthylamino)méthyl]-9,10-éthanoanthracène-9(10H)-éthanol racémique et on isole le R-(−)-α- [(méthylamino)méthyl]-9,10-éthanoanthracène-9(10H)-éthanol, éventuellement sous la forme d'un sel d'addition d'acide, ou

b) on fait réagir un composé de formule II

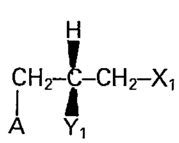 (II),

avec un composé de formule III

$X_2-CH_3$ (III)

où l'un des symboles $X_1$ et $X_2$ représente le groupe amino et l'autre un groupe hydroxy estérifié réactif et $Y_1$ représente un groupe hydroxy libre, et $X_1$ peut également représenter avec $Y_1$ un groupe époxy, et A représente le radical 9,10-éthanoanthracène-9(10H)-yle, ou

c) dans un composé de formule IV

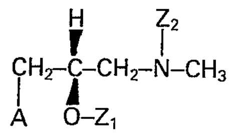 (IV)

où au moins l'un des symboles $Z_1$ et $Z_2$ représente un radical séparable et l'autre éventuellement un hydrogène, ou bien où $Z_1$ et $Z_2$ représentent ensemble un radical bivalent séparable, et A représente le radical 9,10-éthanoanthracène-9(10H)-yle, on sépare le ou les radicaux $Z_1$ et/ou $Z_2$, ou

d) on réduit un composé qui ne se différencie du composé de formule I qu'en ce qu'un atome de carbone voisin de l'atome d'azote est relié à celui-ci par une double liaison ou est substitué par un groupe hydroxy ou par un radical oxo, éventuellement avec un alcoxy inférieur, ou

e) on fixe de l'éthylène sur le R-α-[(méthylamino)méthyl]-9(10H)-anthracène-éthanol, et éventuellement on transforme le R-(−)-α-[(méthylamino)méthyl)-9,10-éthanoanthracène-9(10H)-éthanol ainsi obtenu en un sel d'addition d'acide ou on libère la base à partir d'un sel d'addition d'acide obtenu.

2. Modification du procédé selon la revendication 1, caractérisée en ce qu'on fait réagir un composé de formule générale V

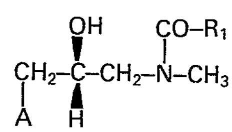 (V)

où R représente un radical hydrocarboné éventuellement substitué ou un radical hétérocyclique éventuellement substitué et A représente un radical 9,10-éthanoanthracène-9(10H)-yle, avec un acide inorganique ou organique fort contenant de l'oxygène ou un halogénure d'un tel acide et on hydrolyse le produit intermédiaire apparu, et si on le désire on transforme le R-(−)-α-[(méthylamino)-méthyl]-9,10-éthanoanthracène-9(10H)-éthanol ainsi obtenu en un sel d'addition d'acide ou on libère la base à partir d'un sel d'addition d'acide obtenu.

3. Procédé selon la revendication 1, caractérisé en ce qu'on prépare un sel d'addition d'acide pharmaceutiquement acceptable du R-(−)-α-[(méthylamino)méthyl]-9,10-éthanoanthracène-9(10H)-éthanol.

4. Procédé selon la revendication 1, caractérisé en ce qu'on prépare le chlorhydrate du R-(−)-α-[(méthylamino)méthyl]-9,10-éthanoanthracène-9(10H)-éthanol.

und seiner Säureadditionssalze, dadurch gekennzeichnet, dass man

a) das racemische α-[(Methylamino)methyl]-9,10-äthanoanthracen-9(10H)-äthanol auftrennt und das R-(−)-α-[(Methylamino)methyl]-9,10-äthanoanthracen-9(10H)-äthanol, gegebenenfalls in Form eines Säureadditionssalzes, isoliert, oder

b) eine Verbindung der Formel II

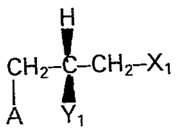
(II),

mit einer Verbindung der Formel III

$$X_2–CH_3 \quad (III)$$

umsetzt, wobei eines der Symbole $X_1$ und $X_2$ die Aminogruppe und das andere eine reaktionsfähige veresterte Hydroxygruppe und $Y_1$ eine freie Hydroxygruppe bedeutet, und $X_1$ auch zusammen mit $Y_1$ eine Epoxygruppe bedeuten kann, und A für den 9,10-Äthanoanthracen-9(10H)-yl-rest steht, oder

c) in einer Verbindung der Formel IV

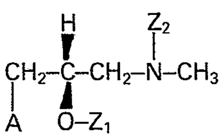
(IV)

in welcher mindestens eines der Symbole $Z_1$ und $Z_2$ einen abspaltbaren Rest und das andere gegebenenfalls Wasserstoff, oder $Z_1$ und $Z_2$ zusammen einen zweiwertigen, abspaltbaren Rest bedeuten, und A für den 9,10-Äthanoanthracen-9(10H)-yl-rest steht, den oder die Reste $Z_1$ und/oder $Z_2$ abspaltet, oder

d) eine Verbindung, welche sich von der Verbindung der Formel I nur dadurch unterscheidet, dass in ihr ein dem Stickstoffatom benachbartes Kohlenstoffatom mit letzterem durch eine Doppelbindung verbunden oder durch eine Hydroxygruppe oder durch einen Oxorest, gegebenenfalls zusammen mit Niederalkoxy, substituiert ist, reduziert, oder

e) an das R-α-[(Methylamino)methyl]-9(10H)-anthracen-äthanol Äthylen anlagert, und gewünschtenfalls das so erhaltene R-(-)-α[Methylamino)methyl]-9,10-äthano-anthracen-9(10H)-äthanol in ein Säureadditionssalz überführt oder aus einem erhaltenen Säureadditionssalz die Base freisetzt.

2. Abänderung des Verfahrens gemäss Anspruch 1, dadurch gekennzeichnet, dass man eine Verbindung der allgemeinen Formel V

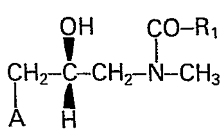
(V)

in welcher R einen gegebenenfalls substituierten Kohlenwasserstoffrest oder einen gegebenenfalls substituierten heterocyclischen Rest und A den 9,10-Äthanoanthracen-9(10H)-yl-rest bedeutet, mit einer starken sauerstoffhaltigen, anorganischen oder organischen Säure oder einem Halogenid einer solchen umsetzt und das entstandene Zwischenprodukt hydrolysiert, und gewünschtenfalls das so erhaltene R-(−)-α-[(Methylamino)methyl]-9,10-äthanoanthracen-9(10H)-äthanol in ein Säureadditionssalz überführt oder aus einem erhaltenen Säureadditionssalz die Base freisetzt.

3. Verfahren nach Anspruch 1, dadurch gekennzeichnet, dass man ein pharmazeutisch annehmbares Säureadditionssalz des R-(−)-α-[(Methylamino)methyl]-9,10-äthanoanthracen-9(10H)-äthanols herstellt.

4. Verfahren nach Anspruch 1, dadurch gekennzeichnet, dass man das Hydrochlorid des R-(−)-α-[(Methylamino)methyl]-9,10-äthanoanthracen-9(10H)-äthanols herstellt.

**Claims**
**(for the Contracting states: BE, CH, DE, FR, GB, IT, LU, NL, SE)**

1. R-(−)-α-[(Methylamino)methyl]-9,10-ethanoanthracene-9(10H)-ethanol of the formula I

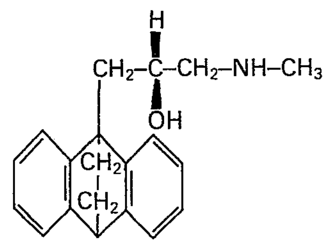
(I)

or an acid addition salt thereof.

2. A pharmaceutically acceptable acid addition salt of the compound of the formula I as indicated in claim 1.

3. The hydrochloride of the compound of the formula I as indicated in claim 1.

4. A process for the production of R-(−)-α-[(methylamino)methyl]-9,10-ethanoanthracene-9(10H)-ethanol of the formula I as indicated in claim 1, or of an acid addition salt thereof, which process comprises.

a) resolving the racemic α-[(methylamino)-methyl]-9,10-ethanoanthracene-9(10H)-ethanol and isolating R-(−)-α-[(methylamino)methyl]-9,10-ethanoanthracene-9(10H)-ethanol, if desired in the form of an acid addition salt, or

b) reacting a compound of the formula II

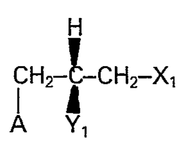
(II),

with a compound of the formula III

$$X_2–CH_3 \quad (III)$$

wherein one of $X_1$ and $X_2$ is the amino group and the other is a reactive esterified hydroxyl group, and $Y_1$ is a free hydroxyl group, and $X_1$ together with $Y_1$ can also be an epopxy group, and A is the 9,10-ethanoanthracen-9(10H)-yl-radical, or

c) in a compound of the formula IV

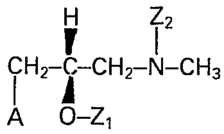

(IV)

in which at least one of $Z_1$ and $Z_2$ is a removable radical and the other may be hydrogen, or $Z_1$ and $Z_2$ together are a divalent removable radical, and A is the 9,10-ethanoanthracen-9(10H)-yl radical, removing $Z_1$ and/or $Z_2$,

d) reducing a compound which differs from the compound of the formula I only in that, in said compound, a carbon atom adjacent to the nitrogen atom is attached to this latter through a double bond or is substituted by a hydroxyl group or an oxo radical, optionally together with lower alkoxy, or

e) adding ethylene to R-α-[(methylamino)methyl]-9(10H)-anthracene-ethanol, or

f) reacting a compound of the general formula V

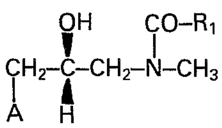

(V)

in which R is an unsubstituted or substituted hydrocarbon radical or an unsubstituted or substituted heterocyclic radical, and A is the 9,10-ethanoanthracen-9(10)-yl radical, with a strong oxygen-containing inorganic or organic acid or with a halide thereof, and hydrolysing the intermediate obtained, and, if desired, converting the resultant R-(−)-α-[(methylamino)methyl]-9,10-ethanoanthracene-9(10H)-ethanol into an acid addition salt and/or liberating the base from a resultant acid addition salt.

5. A pharmaceutical composition containing a compound of the formula I as indicated in claim 1, or a pharmaceutically acceptable acid addition salt thereof, and at least one pharmazeutical carrier.

6. A pharmaceutical composition according to claim 5 in dosage unit form, which contains, per dosage unit, from 2.5 to 50 mg of the compound of the formula I as indicated in claim 1, or of a pharmaceutically acceptable acid addition salt thereof.

7. A pharmaceutical composition according to claim 5 in dosage unit form, which contains, per dosage unit, from 5 to 25 mg of the compound of the formula I as indicated in claim 1, or of a pharmaceutically acceptable acid addition salt thereof.

8. A pharmaceutical composition according to any one of claims 5, 6 or 7, which contains the hydrochloride of the compound of the formula I as indicated in claim 1.

9. Use of the compound of the formula I as indicated in claim 1, or of a pharmaceutically acceptable acid addition salt thereof, as sedative.

10. Use of the compound of the formula I as indicated in claim 1, or of a pharmaceutically acceptable acid addition salt thereof, as antihistamine.

11. Use of the compound of the formula I as indicated in claim 1, or of a pharmaceutically acceptable acid addition salt thereof, as antidepressant.

12. Use of the compound of the formula I as indicated in claim 1, or of a pharmaceutically acceptable acid addition salt thereof, for the preparation of a pharmaceutical composition having sedative, antihistaminic or antidepressive properties.

**Revendications**
**(pour les Etats contractants: BE, CH, DE, FR, GB, IT, LU, NL, SE)**

1. R-(−)-α-[(méthylamino)méthyl]-9,10-éthanoanthracène-9(10H)-éthanol de formule I

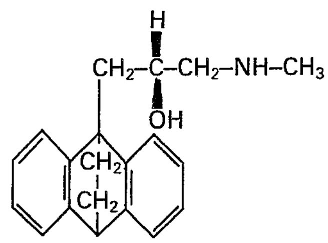

(I)

et ses sels d'addition d'acides.

2. Les sels d'addition d'acides pharmaceutiquement acceptables du composé de formule I donnée dans la revendication 1.

3. Le chlorhydrate du composé de la formule I donnée dans la revendication 1.

4. Procédé de préparation du R-(−)-α-[(méthylamino)méthyl]-9,10-éthanoanthracène-9(10H)-éthanol de formule I donnée dans la revendication 1 et de ses sels d'addition d'acides, caractérisé en ce que

a) on sépare 1'-α-[(méthylamino)méthyl]-9,10-éthanoanthracène-9(10H)-éthanol racémique et on isole le R-(−)-α-[(méthylamino)méthyl]-9,10-éthanoanthracène-9(10H)-éthanol, éventuellement sous la forme d'un sel d'addition d'acide, ou

b) on fait réagir un composé de formule II

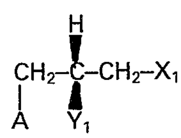

(II),

avec un composé de formule III

$$X_2{-}CH_3 \qquad (III)$$

où l'un des symboles $X_1$ et $X_2$ représente le groupe amino et l'autre un groupe hydroxy estérifié réactif et $Y_1$ un groupe hydroxy libre, et $X_1$ peut également représenter avec $Y_1$ un groupe époxy, et A représente le radical 9,10-éthanoanthracène-9(10H)-yle, ou

c) dans un composé de formule IV

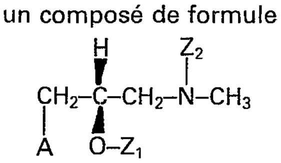

(IV)

où au moins l'un des symboles $Z_1$ ou $Z_2$ repré-

sente un radical séparable et l'autre éventuellement un hydrogène, ou bien où $Z_1$ et $Z_2$ forment ensemble un radical bivalent séparable, et A représente le radical 9,10-éthanoanthracène-9(10H)-yle, on sépare le ou les radicaux $Z_1$ et/ou $Z_2$, ou

d) on réduit un composé qui ne se différencie du composé de formule I qu'en ce qu'un atome de carbone voisin de l'atome d'azote est relié avec celui-ci par une double liaison ou est substitué par un groupe hydroxy ou par un radical oxo, éventuellement avec un alcoxy inférieur, ou

e) on fixe de l'éthylène sur le R-α-[(méthylamino)méthyl]-9(10H)-anthracène-éthanol, ou

f) on fait réagir un composé de formule V

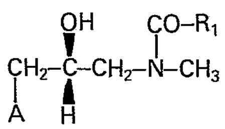
(V)

où R représente un radical hydrocarboné éventuellement substitué ou un radical hétérocyclique éventuellement substitué et A représente un radical 9,10-éthanoanthracène-9(10H)-yle, avec un acide organique ou inorganique fort contenant de l'oxygène ou un halogénure d'un tel acide, et on hydrolyse le produit intermédiaire apparu, et si on le désire on transforme le R-(−)-α-[(méthylamino)-méthyl]-9,10-éthanoanthracène-9(10H)-éthanol ainsi obtenu en un sel d'addition d'acide et/ou on libère le base à partir d'un sel d'addition d'acide obtenu.

5. Préparations pharmaceutiques caractérisées en ce qu'elles contiennent un composé de formule I donnée dans la revendication 1 ou un de ses sels d'addition d'acides pharmaceutiquement acceptables, et au moins un support pharmaceutique.

6. Préparations pharmaceutiques selon la revendication 5 sous forme posologique unitaire, caractérisée en ce qu'elles contiennent par unité posologique de 2,5 à 50 mg du composé de formule I donné dans la revendication 1 ou d'un de ses sels d'addition d'acides pharmaceutiquement acceptables.

7. Préparations pharmaceutiques selon la revendication 5 sous forme posologique unitaire, caractérisées en ce qu'elles contiennent par unité posologique de 5 à 25 mg du composé de formule I donné dans la revendication 1 ou d'un de ses sels d'addition d'acides pharmaceutiquement acceptables.

8. Préparations pharmaceutiques selon l'une des revendications 5, 6 et 7, caractérisées en ce qu'elles contiennent le chlorhydrate du composé de formule I donnée dans la revendication 1.

9. Composé de formule I donnée dans la revendication 1 ou un de ses sels d'addition d'acides pharmaceutiquement acceptables aux fins d'application comme sédatif.

10. Composé de formule I donnée dans la revendication 1 ou un de ses sels d'addition d'acides pharmaceutiquement acceptables aux fins d'application comme anti-histaminique.

11. Composé de formule I donnée dans la revendication 1 ou un de ses sels d'addition d'acides pharmaceutiquement acceptables aux fins d'application comme agent anti-dépresseur.

12. Application du composé de formule I donnée dans la revendication 1 ou d'un de ses sels d'addition d'acides pharmaceutiquement acceptables à la préparation de préparations pharmaceutiques à action sédative, anti-histaminique et anti-dépressive.

**Claims for the designated state: AT**

1. A process for the production of R-(−)-α-[(methylamino)methyl]-9,10-ethanoanthracene-9(10H)-ethanol of the formula I or of an acid addition salt thereof,

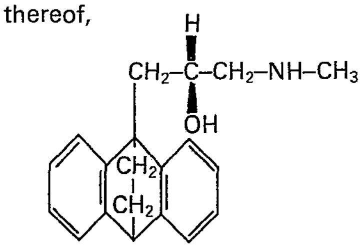
(I)

which process comprises

a) resolving the racemic α-[(methylamino)-methyl]-9,10-ethanoanthracene-9(10H)-ethanol and isolating R-(−)-α-[(methylamino)methyl]-9,10-ethanoanthracene-9(10)-ethanol, if desired in the form of an acid addition salt, or

b) reacting a compound of the formula II

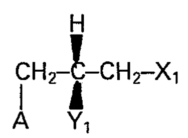
(II),

with a compound of the formula III

$$X_2-CH_3 \qquad (III)$$

wherein one of $X_1$ and $X_2$ is the amino group and the other is a reactive esterified hydroxyl group, and $Y_1$ is a free hydroxyl group, and $X_1$ together with $Y_1$ can also be an epoxy group, and A is the 9,10-ethanoanthracene-9(10H)-yl radical, or

c) in a compound of the formula IV

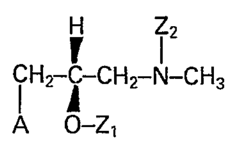
(IV)

in which at least one of $Z_1$ and $Z_2$ is a removable radical and the other may be hydrogen, or $Z_1$ and $Z_2$ together are a divalent removable radical, and A is the 9,10-ethanoanthracene-9(10H)- yl radical, removing $Z_1$ and/or $Z_2$.

d) reducing a compound which differs from the compound of the formula I only in that, in said compound, a carbon atom adjacent to the nitrogen atom is attached to this latter through a double bond or is substituted by a hydroxyl group or an oxo radical, optionally together with lower alkoxy, or

e) adding ethylene to R-α-[(methylamino)-methyl]-9(10H)-anthracene-ethanol, and, if desired, converting the resultant R-(−)-α-[(methylamino)methyl]-9,10-ethanoanthracene-9(10H)-ethanol into an acid addition salt and/or liberating the base from a resultant acid addition sald.

2. A modification of the process according to claim 1, which comprises reacting a compound of the general formula V

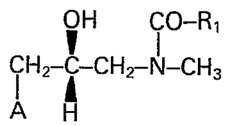

(V)

in which $R_1$ is an unsubstituted or substituted hydrocarbon radical or an unsubstituted or substituted heterocyclic radical, and A is the 9,10-ethanoanthracene-9(10H)-yl radical, with a strong oxygen-containing inorganic or organic acid or with a halide thereof, and hydrolysing the intermediate obtained, and, if desired, converting the resultant R-(−)-α-[(methylamino)methyl]-9,10-ethanoanthracene-9(10H)-ethanol into an acid addition salt and/or liberating the base from a resultant acid addition salt.

3. A process according to claim 1 for the production of a pharmaceutically acceptable acid addition salt of R-(−)-α-[(methylamino)methyl]-9,10-ethanoanthracene-9(10H)-ethanol.

4. A process according to claim 1 for the production of the hydrochloride of R-(−)-α-[(methylamino)methyl]-9,10-ethanoanthracene-9(10H)-ethanol.

**Revendications pour l'Etat contractant: AT**

1. Procédé de préparation du R-(−)-α-[méthylamino)méthyl]-9,10-éthanoanthracène-9(10H)-éthanol de formule I

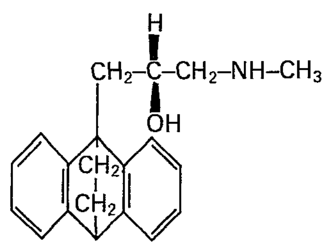

(I)

et de ses sels d'addition d'acides, caractérisé en ce que

a) on sépare 1'α-[(méthylamino)méthyl]-9,10-éthanoanthracène-9(10H)-éthanol racémique et on isole le R-(−)-α- [(méthylamino)méthyl]-9,10-éthanoanthracène-9(10H)-éthanol, éventuellement sous la forme d'un sel d'addition d'acide, ou

b) on fait réagir un composé de formule II

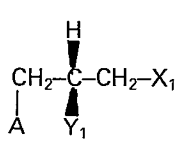

(II),

avec un composé de formule III

$$X_2{-}CH_3 \qquad (III)$$

où l'un des symboles $X_1$ et $X_2$ représente le groupe amino et l'autre un groupe hydroxy estérifié réactif et $Y_1$ représente un groupe hydroxy libre, et $X_1$ peut également représenter avec $Y_1$ un groupe époxy, et A représente le radical 9,10-éthanoanthracène-9(10H)-yle, ou

c) dans un composé de formule IV

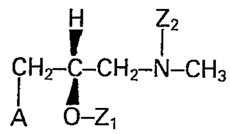

(IV)

où au moins l'un des symboles $Z_1$ et $Z_2$ représente un radical séparable et l'autre éventuellement un hydrogène, ou bien où $Z_1$ et $Z_2$ représentent ensemble un radical bivalent séparable, et A représente le radical 9,10-éthanoanthracène-9(10H)-yle, on sépare le ou les radicaux $Z_1$ et/ou $Z_2$, ou

d) on réduit un composé qui ne se différencie du composé de formule I qu'en ce qu'un atome de carbone voisin de l'atome d'azote est relié à celui-ci par une double liaison ou est substitué par un groupe hydroxy ou par un radical oxo, éventuellement avec un alcoxy inférieur, ou

e) on fixe de l'éthylène sur le R-α-[(méthylamino)méthyl]-9(10H)-anthracène-éthanol, et éventuellement on transforme le R-(−)-α-[(méthylamino)méthyl)-9,10-éthanoanthracène-9(10H)-éthanol ainsi obtenu en un sel d'addition d'acide ou on libère la base à partir d'un sel d'addition d'acide obtenu.

2. Modification du procédé selon la revendication 1, caractérisée en ce qu'on fait réagir un composé de formule générale V

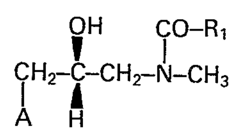

(V)

où R représente un radical hydrocarboné éventuellement substitué ou un radical hétérocyclique éventuellement substitué et A représente un radical 9,10-éthanoanthracène-9(10H)-yle, avec un acide inorganique ou organique fort contenant de l'oxygène ou un halogénure d'un tel acide et on hydrolyse le produit intermédiaire apparu, et si on le désire on transforme le R-(−)-α-[(méthylamino)-méthyl]-9,10-éthanoanthracène-9(10H)-éthanol ainsi obtenu en un sel d'addition d'acide ou on libère la base à partir d'un sel d'addition d'acide obtenu.

3. Procédé selon la revendication 1, caractérisé en ce qu'on prépare un sel d'addition d'acide pharmaceutiquement acceptable du R-(−)-α-[(méthylamino)méthyl]-9,10-éthanoanthracène-9(10H)-éthanol.

4. Procédé selon la revendication 1, caractérisé en ce qu'on prépare le chlorhydrate du R-(−)-α-[(méthylamino)méthyl]-9,10-éthanoanthracène-9(10H)-éthanol.